(19) Europäisches Patentamt
European Patent Office
Office européen des brevets

(11) Veröffentlichungsnummer: **0 017 094**
**B1**

(12) **EUROPÄISCHE PATENTSCHRIFT**

(45) Veröffentlichungstag der Patentschrift: **25.11.81**

(51) Int. Cl.³: **C 07 J 1/00**, A 61 K 31/565 // C07J21/00

(21) Anmeldenummer: **80101470.5**

(22) Anmeldetag: **20.03.80**

(54) **11-Methylensteroide, Verfahren zu ihrer Herstellung und diese enthaltende pharmazeutische Präparate.**

(30) Priorität: **30.03.79 DE 2913381**

(43) Veröffentlichungstag der Anmeldung: **15.10.80 Patentblatt 80/21**

(45) Bekanntmachung des Hinweises auf die Patenterteilung: **25.11.81 Patentblatt 81/47**

(84) Benannte Vertragsstaaten: **AT BE CH DE FR GB IT LU NL SE**

(56) Entgegenhaltungen:
**DE-A-1 934 272**
**DE-A-2 361 120**
**DE-B-1 793 641**
**RECUEIL JOURNAL OF THE ROYAL NETHERLANDS CHEMICAL SOCIETY, Band 94(2), Februar 1975.**
**'s Gravenhage, NL, A. J. VAN DEN BROEK et al.: »11-Alkylidene steroids in the 19-nor series«, Seiten 35 – 39**

(73) Patentinhaber: **SCHERING AKTIENGESELLSCHAFT**
**Berlin und Bergkamen,**
**Müllerstrasse 170/178 Postfach 65 0311,**
**D-1000 Berlin 65 (DE)**

(72) Erfinder: **Hofmeister, Helmut, Dr., Weislingenstrasse 4, D-1000 Berlin 28 (DE)**
Erfinder: **Wiechert, Rudolf, Prof. Dr., Petzowerstrasse 8 A, D-1000 Berlin 39 (DE)**
Erfinder: **Annen, Klaus, Dr., Seegefelderstrasse 194, D-1000 Berlin 20 (DE)**
Erfinder: **Laurent, Henry, Dr., Glambecker Weg 21, D-1000 Berlin 28 (DE)**
Erfinder: **Steinbeck, Hermann, Dr., Hammerstrasse 46, D-1000 Berlin 37 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 017 094 B1**

BUNDESDRUCKEREI BERLIN

# 11-Methylensteroide, Verfahren zu ihrer Herstellung und diese enthaltende pharmazeutische Präparate

Die Erfindung betrifft 11-Methylensteroide, Verfahren zu ihrer Herstellung und diese enthaltende pharmazeutische Präparate.

17α-Chlorethinyl-18-methyl-$\Delta^4$-östrene ohne Methylengruppe in 11-Stellung sind bereits bekannt, zum Beispiel als 3-Oxoverbindungen aus der deutschen Auslegeschrift 1 793 641 und als 3-Desoxoverbindungen aus der deutschen Offenlegungsschrift 1 934 272. Obwohl die 17α-Chlorethinylverbindungen ein ähnliches Wirkungsspektrum besitzen wie die entsprechenden 17α-Ethinylverbindungen, haben die 17α-Chlorethinylverbindungen niemals die Bedeutung der 17α-Ethinylverbindungen erlangt. So sind in den Beispielen der deutschen Offenlegungsschrift 1 934 272 auch nur die 17α-Ethinylverbindungen beschrieben worden. Im Gegensatz zu den 17α-Ethinylverbindungen sind die 17α-Chlorethinylverbindungen niemals in Arznei- oder Kontrazeptionsmitteln verwendet worden.

In der neuen Gruppe der 11-Methylensteroide sind die 17-Chlorethinylsteroide nicht beschrieben und auch nicht mehr erwähnt worden. In der deutschen Offenlegungsschrift 2 361 120 werden zum Beispiel 11-Methylen-17α-ethinyl-18-methyl-$\Delta^4$-östrene beschrieben, die eine starke gestagene Wirkung zeigen.

Die folgende Tabelle zeigt die überlegene Wirkung von 17α-Chlorethinyl-17β-hydroxy-18-methyl-11-methylen-4-östren-3-on (A) im Vergleich zu 17α-Ethinyl-17β-hydroxy-18-methyl-11-methylen-4-östren-3-on (B) im Ovulationshemmtest an Ratten.

Ovulationshemmung bei Ratten nach subkutaner Applikation

| Dosis (mg) | Verbindung A (% Hemmung) | Verbindung B (% Hemmung) |
|---|---|---|
| 0,1 | 100 | 100 |
| 0,03 | 83 | 0 |

Die Ovulationshemmung wurde durch Tubeninspektion ermittelt.

Aus der Tabelle ist ersichtlich, daß die Chlorethinylverbindung A mindestens 3mal stärker wirkt als die Ethinylverbindung B. Darüber hinaus besitzt A geringere androgene Nebenwirkung als B.

Die höheren Ester der erfindungsgemäßen Chlorethinylverbindungen zeichnen sich durch protrahierte Wirksamkeit aus.

Aufgrund der starken ovulationshemmenden Wirkung können die erfindungsgemäßen Verbindungen besonders vorteilhaft in Antikonzeptionspräparaten Verwendung finden, wobei sie als Gestagenkomponente in Kombination mit einer östrogen wirksamen Hormonkomponente, wie zum Beispiel Äthinylöstradiol, oder als alleinige Wirkkomponente eingesetzt werden. Die Verbindungen können aber auch in Präparaten zur Behandlung gynäkologischer Störungen eingesetzt werden.

Zum Gebrauch werden die neuen Verbindungen mit den in der galenischen Pharmazie üblichen Zusätzen, Trägersubstanzen und Geschmackskorrigentien nach an sich bekannten Methoden zu den üblichen Arzneimittelformen verarbeitet. Für die orale Applikation kommen insbesondere Tabletten, Dragées, Kapseln, Pillen, Suspensionen oder Lösungen in Frage. Für die parenterale Applikation kommen insbesondere ölige Lösungen, wie zum Beispiel Sesamöl- oder Rizinusöllösungen in Frage, die gegebenenfalls zusätzlich noch ein Verdünnungsmittel, wie zum Beispiel Benzylbenzoat oder Benzylalkohol, enthalten können. Die Konzentration des Wirkstoffes ist abhängig von der Applikationsform. So enthalten beispielsweise Tabletten zur oralen Applikation vorzugsweise 0,01—0,5 mg Wirkstoff und Lösungen zur parenteralen Applikation vorzugsweise 1—100 mg Wirkstoff pro 1 ml Lösung.

Die Dosierung der erfindungsgemäßen pharmazeutischen Präparate kann sich mit der Form und dem Zweck der Verabfolgung ändern. Beispielsweise liegt die tägliche kontrazeptive Dosis bei oraler Applikation bei 0,05 bis 0,5 mg Wirkstoff gemäß Formel I.

Die Steroide der allgemeinen Formal I gemäß Anspruch 1 enthalten in 17β-Stellung die Gruppierung OR, worin R ein Wasserstoffatom oder eine Acylgruppe bedeutet. Als Acylgruppen R kommen solche von physiologisch verträglichen Säuren in Frage. Bevorzugte Säuren sind organische Carbon- und Sulfonsäuren mit 1—17 Kohlenstoffatomen, die der aliphatischen, cycloaliphatischen, aromatischen, aromatisch-aliphatischen oder heterocyclischen Reihe angehören. Diese Säuren können auch gesättigt oder ungesättigt, ein- oder mehrbasisch und/oder in üblicher Weise substituiert sein. Als Beispiele für die Substituenten seien Hydroxy-, Alkoxy-, Acyloxy-, Oxo-, Aminogruppen oder Halogenatome genannt.

Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure,

Pelargonsäure, Caprinsäure, Undecylsäure, Laurinsäure, Tridecylsäure, Myristinsäure, Pentadecylsäure, Trimethylessigsäure, Diäthylessigsäure, tert.-Butylessigsäure, $\beta$-Cyclopentylpropionsäure, Cyclohexylessigsäure, Cyclohexancarbonsäure, Phenylessigsäure, Phenoxyessigsäure, Mono-, Di- und Trichloressigsäure, Aminoessigsäure, Diäthylaminoessigsäure, Piperidinoessigsäure, Morpholinoessigsäure, Milchsäure, Bernsteinsäure, Adipinsäure, Benzoesäure, Nikotinsäure, Isonikotinsäure, Furan-2-carbonsäure, O-Tridecanoylglykolsäure, O-Hexadecanoylglykolsäure, $\beta$-Tridecanoyloxypropionsäure. Als Sulfonsäuren seien genannt: Methan-, Äthan-, $\beta$-Chloräthan-, Propan-, Isopropan-, Butan-, Cyclopentan-, Cyclohexan-, Benzol-, p-Toluol-, p-Chlorbenzolsulfonsäure, ferner N,N-Dimethyl-, N,N-Diäthyl-, Bis($\beta$-Chloräthyl)-aminosulfonsäure, Pyrrolidino-, Piperidino-, Piperazino-, N-Methylpiperazino- und Morpholinosulfonsäure.

Das in Anspruch 9 gekennzeichnete Verfahren wird nach an sich bekannten Methoden mit einer metallorganischen Chlorethinylverbindung durchgeführt, indem man das 17-Oxosteroid in einem geeigneten Lösungsmittel mit der metallorganischen Chlorethinylverbindung umsetzt. Die Chlorethinylverbindung wird in situ aus 1,2-Dichlorethylen und einer etherischen Alkyllithiumlösung, wie zum Beispiel Methyl- oder Butyllithiumlösung, gebildet. Geeignete Lösungsmittel sind Tetrahydrofuran und Diethylether.

Bei Vorliegen einer 3-Ketogruppe wird diese zweckmäßigerweise vor der Chlorethinylierung geschützt. Die Schutzgruppe soll durch saure Hydrolyse abspaltbar sein. Die Schutzgruppe Y soll mit der (den) Doppelbindung(en), welche im Ring A oder B vorhanden ist bzw. sind, eine solche Anordnung von Atomen bilden, daß die Verbindung durch saure Hydrolyse zu einem 4,5-ungesättigten Keton umgewandelt wird. In einer bevorzugten Ausführungsform wird die Ketogruppe durch Ketalbildung geschützt. Die Ketalreste leiten sich von den üblicherweise zum Schutz freier Oxogruppen verwendeten Alkoholen und Thioalkoholen ab, beispielsweise genannt seien: Ethylenglykol, 2,2-Dimethyl-propandiol-(1,3) und Ethandithiol-(1,2). Die 3-Ketogruppe kann aber auch durch Enoläther-, Enolester- oder Enaminbildung partiell geschützt werden.

Die Abspaltung der 3-Ketoschutzgruppe, die vor oder auch nach der möglichen Veresterung erfolgen kann, wird nach den dem Fachmann bekannten Methoden durchgeführt. Zur Spaltung kommen beispielsweise Mineralsäuren, wie zum Beispiel Perchlorsäure, Schwefelsäure oder Salzsäure, oder organische Säuren, wie zum Beispiel Oxalsäure, in Betracht. Die Spaltung wird vorzugsweise in alkoholischer Lösung oder in anderen polaren Lösungsmitteln, wie zum Beispiel Aceton, bei Temperaturen zwischen etwa 20 und 100° C durchgeführt.

Für die sich gegebenenfalls anschließende Veresterung kommen die üblicherweise in der Steroidchemie zur Veresterung angewendeten Verfahren in Frage. Beispielsweise sei die Umsetzung mit Säuren oder Säureanhydriden in Gegenwart starker Säuren, wie zum Beispiel Trifluoressigsäure oder p-Toluolsulfonsäure, bei Raumtemperatur oder etwas angehobener Temperatur; oder die Umsetzung mit einem Säureanhydrid in Gegenwart eines tertiären Amins in der Wärme bei etwa 20—200° C genannt.

Werden Pyridin und 4-(Dimethylamino)-pyridin als tertiäre Amine gemeinsam angewandt, kann die Veresterung bei Raumtemperatur durchgeführt werden.

Die nach dem erfindungsgemäßen Verfahren gemäß Anspruch 9 eingesetzten Ausgangsverbindungen der allgemeinen Formel II werden in der deutschen Offenlegungsschrift 2 361 120 beschrieben oder können analog den dort angegebenen Vorschriften hergestellt werden.

Ein bevorzugtes Ausgangsmaterial 3,3-(2',2'-Dimethyltrimethylendioxy)-18-methyl-11-methylen-5-östren-17-on kann zum Beispiel wie folgt hergestellt werden:

1,0 g 18-Methyl-11-methylen-4-östren-3,17-dion in 10 ml Methylenchlorid werden bei Raumtemperatur mit 1 ml o-Ameisensäuretriethylester, 2,0 g 2,2-Dimethylpropandiol-(1,3) und 5 mg p-Toluolsulfonsäure versetzt. Nach 5 Stunden verdünnt man mit Methylenchlorid, wäscht neutral und trocknet. Das Rohprodukt wird an Kieselgel mit Hexan-Aceton (0—3%) chromatographiert. Es werden 800 mg 3,3-(2',2'-Dimethyl-trimethylendioxy)-18-methyl-11-methylen-5-östren-17-on vom Schmelzpunkt 194° C erhalten. $[\alpha]_D = +105{,}7°$.

## Beispiel 1

### a) 17$\alpha$-Chlorethinyl-3,3-(2',2'-dimethyl-trimethylendioxy)-18-methyl-11-methylen-5-östren-17$\beta$-ol

Zu 2,5 ml 1,2-Dichlorethylen in 15 ml absolutem Ether werden unter Argon bei —0° C 12,5 ml einer 5%igen etherischen Methyllithiumlösung getropft. Nach 30 Minuten gibt man 1,0 g 3,3-(2',2'-Dimethyltrimethylendioxy)-18-methyl-11-methylen-5-östren-17-on in 30 ml absolutem Ether hinzu, rührt weitere 20 Minuten, versetzt mit gesättigter Ammoniumchloridlösung, verdünnt mit Ether und wäscht die Lösung mit Wasser. Das Rohprodukt wird aus Aceton/Hexan umkristallisiert. Es werden 740 mg 17$\alpha$-Chlorethinyl-3,3-(2',2'-dimethyltrimethylendioxy)-18-methyl-11-methylen-5-östren-17$\beta$-ol vom Schmelzpunkt 214,0° C erhalten. $[\alpha]_D = -16{,}8°$.

b) 17α-Chlorethinyl-17β-hydroxy-18-methyl-11-methylen-4-östren-3-on

1,0 g 17α-Chlorethinyl-3,3-(2',2'-dimethyl-trimethylendioxy)-18-methyl-11-methylen-5-östren-17β-ol in 40 ml Aceton werden bei Raumtemperatur mit 0,5 ml halbkonzentrierter Salzsäure versetzt. Nach 1,5 Stunden neutralisiert man mit Natriumhydrogencarbonatlösung, engt das Gemisch im Vakuum weitgehend ein und löst den Rückstand in Essigester. Das Rohprodukt wird durch präparative Schichtchromatographie (Laufmittel: Hexan/Essigester 7 : 3) gereinigt. Es werden 270 mg 17α-Chorethinyl-17β-hydroxy-18-methyl-11-methylen-4-östren-3-on erhalten. Schmelzpunkt 168,9° C. $[\alpha]_D = +35,6°$.

Beispiel 2

17β-Acetoxy-17α-chlorethinyl-18-methyl-11-methylen-4-östren-3-on

1,0 g 17α-Chlorethinyl-17β-hydroxy-18-methyl-11-methylen-4-östren-3-on werden in 10 ml Pyridin mit 5 ml Acetanhydrid und 50 mg 4-(Dimethylamino)-pyridin 3 Stunden unter Argon bei Raumtemperatur gerührt. Die Lösung wird in schwefelsäurehaltiges Eis/Wasser gegeben. Das ausgefallene Produkt wird abgesaugt, in Essigester gelöst, mit Wasser gewaschen und getrocknet. Nach Chromatographieren des Rohproduktes an Kieselgel mit Aceton/Hexan werden 710 mg 17β-Acetoxy-17α-chlorethinyl-18-methyl-11-methylen-4-östren-3-on als schaumiges Produkt erhalten.

Beispiel 3

17α-Chlorethinyl-18-methyl-11-methylen-17β-(O-tridecanoyl-glykoloyloxy)-4-östren-3-on

Zu 200 mg 17α-Chlorethinyl-17β-hydroxy-18-methyl-11-methylen-4-östren-3-on und 200 mg 4-(Dimethylamino)-pyridin in 2 ml Tetrachlorethylen werden bei 100° C unter Argon innerhalb 2 Stunden 650 mg O-Tridecanoylglykoloylchlorid getropft. Man erhitzt 3 Stunden bei 80° C, läßt abkühlen, verdünnt mit Ether und wäscht mit wäßriger Oxalsäurelösung und Wasser neutral. Das Rohprodukt wird an Kieselgel mit Aceton/Hexan chromatographiert. Man erhält 210 mg 17α-Chlorethinyl-18-methyl-11-methylen-17β-(O-tridecanoyl-glykoloyloxy)-4-östren-3-on als farbloses Öl.

Beispiel 4

17α-Chlorethinyl-18-methyl-11-methylen-4-östren-17β-ol

Zu 1,3 ml 1,2-Dichlorethylen in 10 ml absolutem Ether werden unter Argon bei 0° C 7 ml einer 5%igen etherischen Methyllithiumlösung getropft. Nach 30 Minuten gibt man 510 mg 18-Methyl-11-methylen-4-östren-17-on in 12 ml absolutem Ether hinzu, rührt weitere 15 Minuten, versetzt mit gesättigter Ammoniumchloridlösung, verdünnt mit Ether und wäscht die Lösung mit Wasser. Das Rohprodukt wird an Kieselgel mit Aceton/Hexan chromatographiert. Es werden 360 mg 17α-Chlorethinyl-18-methyl-11-methylen-4-östren-17β-ol als Öl erhalten. Eine aus Aceton/Hexan kristallisierte Probe schmilzt bei 165,5° C. $[\alpha]_D = +17,6°$.

Beispiel 5

17β-Acetoxy-17α-chlorethinyl-18-methyl-11-methylen-4-östren

210 mg 17α-Chlorethinyl-18-methyl-11-methylen-4-östren-17β-ol in 3 ml Pyridin werden bei Raumtemperatur mit 10 mg 4-(Dimethylamino)-pyridin und 1,5 ml Acetanhydrid umgesetzt. Die Lösung wird in schwefelsäurehaltiges Eis/Wasser gegeben. Nach Extraktion mit Essigester wird die Lösung mit Wasser gewaschen und getrocknet. Das Rohprodukt wird durch präparative Schichtchromatographie (Laufmittel: Hexan/Essigester 7 : 3) gereinigt. Es werden 130 mg 17β-Acetoxy-17α-chlorethinyl-18-methyl-11-methylen-4-östren als schaumiges Produkt isoliert.

Beispiel 6

17α-Chlorethinyl-18-methyl-11-methylen-17β-undecanoyloxy-4-östren

Aus einer Lösung von 400 mg Undecylsäure in 40 ml Benzol werden 6 ml abdestilliert. Nach dem Abkühlen auf Raumtemperatur gibt man 0,4 ml Trifluoressigsäureanhydrid hinzu, rührt 30 Minuten und

setzt 300 mg 17α-Chlorethinyl-18-methyl-11-methylen-4-östren-17β-ol zu. Nach 2 Stunden wird das Gemisch mit 5 ml Aceton/Wasser (1 : 1) versetzt, 30 Minuten gerührt und anschließend im Vakuum eingeengt. Der Rückstand wird in Methylenchlorid aufgenommen, mit Natriumhydrogencarbonatlösung und Wasser gewaschen und getrocknet. Das Rohprodukt wird durch präparative Schichtchromatographie (Laufmittel: Hexan/Essigester 7 : 3) gereinigt. Es werden 115 mg 17α-Chlorethinyl-18-methyl-11-methylen-17β-undecanoyloxy-4-östren als öliges Produkt erhalten.

**Patentansprüche für die Vertragsstaaten: BE, CH, DE, FR, GB, IT, LU, NL, SE**

1. 11-Methylensteroide der allgemeinen Formel I

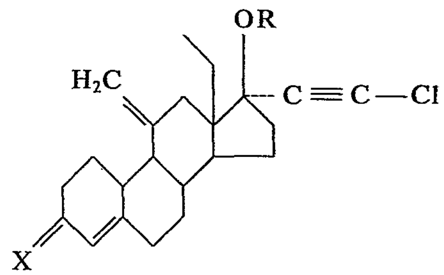

(I)

worin

R ein Wasserstoffatom oder eine Acylgruppe und
X ein Sauerstoffatom oder zwei Wasserstoffatome

bedeuten.

2. 17α-Chlorethinyl-17β-hydroxy-18-methyl-11-methylen-4-östren-3-on.
3. 17β-Acetoxy-17α-chlorethinyl-18-methyl-11-methylen-4-östren-3-on.
4. 17α-Chlorethinyl-18-methyl-11-methylen-17β-(O-tridecanoylglykoloyloxy)-4-östren-3-on.
5. 17α-Chlorethinyl-18-methyl-11-methylen-4-östren-17β-ol.
6. 17β-Acetoxy-17α-chlorethinyl-18-methyl-11-methylen-4-östren.
7. 17α-Chlorethinyl-18-methyl-11-methylen-17β-undecanoyloxy-4-östren.
8. Pharmazeutisches Präparat, gekennzeichnet durch einen Gehalt eines Steroids nach einem der Ansprüche 1—7.
9. Verfahren zur Herstellung von 11-Methylensteroiden der allgemeinen Formel I

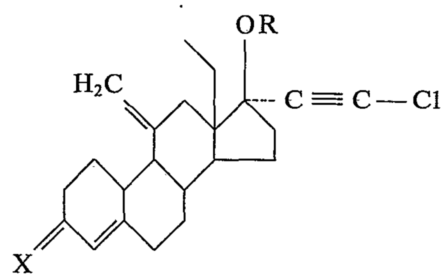

(I)

worin

R ein Wasserstoffatom oder eine Acylgruppe und
X ein Sauerstoffatom oder zwei Wasserstoffatome

bedeuten,
dadurch gekennzeichnet, daß man ein 11-Methylen-17-oxo-Steroid der allgemeinen Formel II

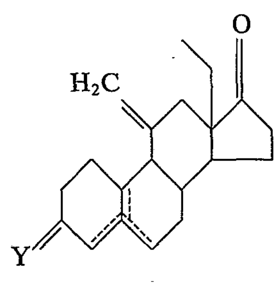

(II)

worin

Y eine freie Oxogruppe oder eine säurehydrolysierbare Oxoschutzgruppe oder zwei Wasserstoffatome darstellt und

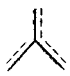 eine Doppelbindung in 4,5-, 5,6- oder 5,10-Stellung oder zwei von 3- und 5-Stellung ausgehende Doppelbindungen bedeutet,

nach an sich bekannten Methoden mit 1,2-Dichlorethylen und einer etherischen Alkyllithiumlösung zu einer Verbindung der allgemeinen Formel I, worin R ein Wasserstoffatom bedeutet, umsetzt, eine primär eingeführte 3-Oxoschutzgruppe hydrolysiert und, je nach der letztlich gewünschten Bedeutung von R, gegebenenfalls die 17-Hydroxygruppe vor oder nach der Abspaltung der Schutzgruppe verestert.

**Patentanspruch für den Vertragsstaat: AT**

Verfahren zur Herstellung von 11-Methylensteroiden der allgemeinen Formel I

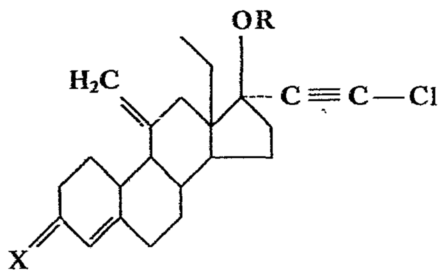

(I)

worin

R ein Wasserstoffatom oder eine Acylgruppe und
X ein Sauerstoffatom oder zwei Wasserstoffatome

bedeuten,
dadurch gekennzeichnet, daß man ein 11-Methylen-17-oxo-Steroid der allgemeinen Formel II

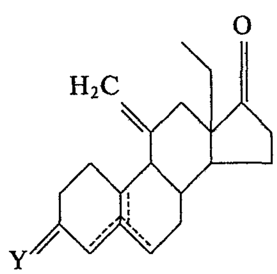

(II)

worin

Y eine freie Oxogruppe oder eine säurehydrolysierbare Oxoschutzgruppe oder zwei Wasserstoffatome darstellt und

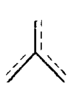 eine Doppelbindung in 4,5-, 5,6- oder 5,10-Stellung oder zwei von 3- und 5-Stellung ausgehende Doppelbindungen bedeutet,

nach an sich bekannten Methoden mit 1,2-Dichlorethylen und einer etherischen Alkyllithiumlösung zu einer Verbindung der allgemeinen Formel I, worin R ein Wasserstoffatom bedeutet, umsetzt, eine primär eingeführte 3-Oxoschutzgruppe hydrolysiert und, je nach der letztlich gewünschten Bedeutung von R, gegebenenfalls die 17-Hydroxygruppe vor oder nach der Abspaltung der Schutzgruppe verestert.

**Claims for the Contracting states: BE, CH, DE, FR, GB, IT, LU, NI, SE**

1. 11-methylene steroids of the general formula I

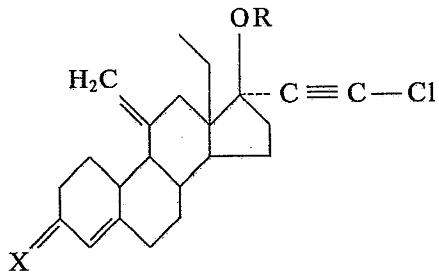

(I)

in which

R denotes a hydrogen atom or an acyl group and
X denotes an oxygen atom or two hydrogen atoms.

2. 17α-chloroethynyl-17β-hydroxy-18-methyl-11-methylene-4-oestren-3-one.
3. 17β-acetoxy-17α-chloroethynyl-18-methyl-11-methylene-4-oestren-3-one.
4. 17α-chloroethynyl-18-methyl-11-methylene-17β-(O-tridecanoylglycoloyloxy)-4-oestren-3-one.
5. 17α-chloroethynyl-18-methyl-11-methylene-4-oestren-17β-ol.
6. 17β-acetoxy-17α-chloroethynyl-18-methyl-11-methylene-4-oestrene.
7. 17α-chloroethynyl-18-methyl-11-methylene-17β-undecanoyloxy-4-oestrene.
8. Pharmaceutical preparation, characterised in that it contains a steroid according to one of claims 1—7.
9. Process for the manufacture of 11-methylene steroids of the general formula I

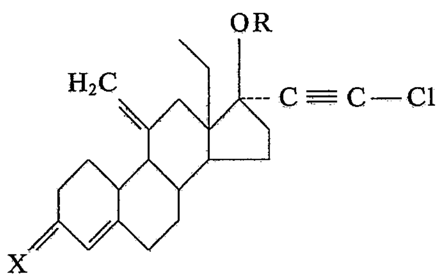

(I)

in which

R denotes a hydrogen atom or an acyl group and
X denotes an oxygen atom or two hydrogen atoms,

characterised in that, an 11-methylene-17-oxo-steroid of the general formula II

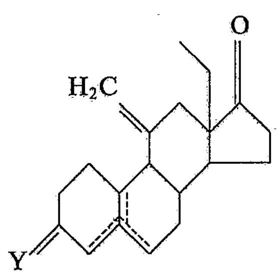

(II)

in which

Y denotes a free oxo group or an acid-hydrolysable oxo-protecting group or two hydrogen atoms and

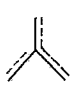 denotes a double bond in the 4,5-, 5,6- or 5,10-position or two double bonds extending from the 3- and 5-positions,

is reacted, according to methods known per se, with 1,2-dichloroethylene and an ethereal alkyllithium solution to form a compound of the general formula I, in which R denotes a hydrogen atom, a 3-oxo-protecting group originally introduced is hydrolysed and, depending on the finally desired meaning of R, optionally the 17-hydroxy group is esterified before or after the protecting group is split off.

**Claim for the Contracting statei AT**

Process for the manufacture of 11-methylene steroids of the general formula I

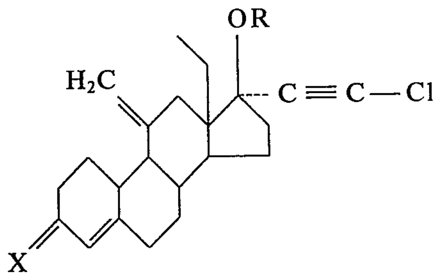

(I)

in which

R denotes a hydrogen atom or an acyl group and
X denotes an oxygen atom or two hydrogen atoms,

characterised in that, an 11-methylene-17-oxo-steroid of the general formula II

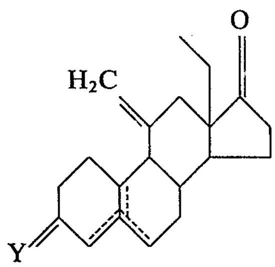

(II)

in which

Y denotes a free oxo group or an acid-hydrolysable oxo-protecting group or two hydrogen atoms and

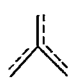 denotes a double bond in the 4,5-, 5,6- or 5,10-position or two double bonds extending from the 3- and 5-positions,

is reacted, according to methods known per se, with 1,2-dichloroethylene and an ethereal alkyllithium solution to from a compound of the general formula I, in which R denotes a hydrogen atom, a 3-oxo-protecting group originally introduced is hydrolysed and, depending on the finally desired meaning of R, optionally the 17-hydroxy group is esterified before or after the protecting group is split off.

**Revendications pour les Etates contractants: BE, CH, DE, FR, GB, IT, LU, NL, SE**

1. Méthylène-11 stéroïdes répondant à la formule générale I

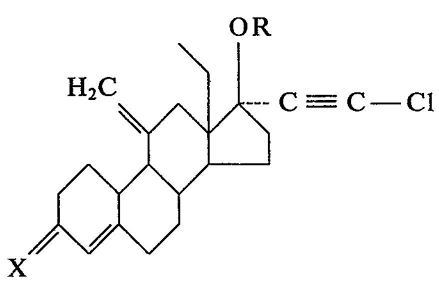

(I)

dans laquelle

R représente un atome d'hydrogène ou un radical acyle et
X représente un atome d'oxygène ou deux atomes d'hydrogène.

2. Chloréthynyl-17α hydroxy-17β méthyl-18 méthylène-11 oestrène-4 one-3.
3. Acétoxy-17β chloréthynyl-17α méthyl-18 méthylène-11 oestrène-4 one-3.
4. Chloréthynyl-17α méthyl-18 méthylène-11 (0-tridécanoyl-glycoloyloxy)-17β oestrène-4 one-3.
5. Chloréthynyl-17α méthyl-18 méthylène-11 oestrène-4 ol-17β.
6. Acétoxy-17β chloréthynyl-17α méthyl-18 méthylène-11 oestrène-4.
7. Chloréthynyl-17α méthyl-18 méthylène-11 undécanoyloxy-17β oestrène-4.
8. Médicaments renfermant un stéroide selon l'une quelconque des revendications 1 à 7.
9. Procédé de préparation de méthylène-11 stéroides répondant à lá formule générale I

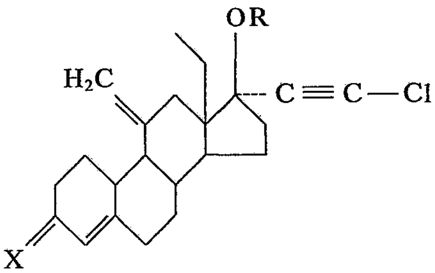

(I)

procédé caractérisé en ce qu'on fait réagir un méthylène-11 oxo-17 stéroïde répondant à la formule générale II

dans laquelle

R représente un atome d'hydrogène ou un radical acyle et
X représente un atome d'oxygène ou deux atomes d'hydrogène,

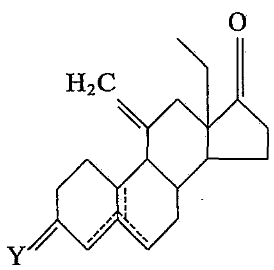

(II)

dans laquelle

Y représente un groupe oxo libre ou protégé par un groupe hydrolysable en milieu acide ou représente deux atomes d'hydrogène et

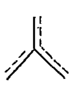 représente une double liaison en position 4,5, 5,6 ou 5,10 ou deux doubles liaisons partant de la position 3 et de la position 5,

par des méthodes connues, avec le dichloro-1,2 éthylène et une solution éthérée d'un alkyl-lithium, de manière à obtenir un composé de formule générale I dans lequel R représente un atome d'hydrogène, on hydrolyse un groupe protecteur de la fonction cétonique en 3 dans le cas où l'on a préalablement introduit un tel groupe et, suivant la signification que doit avoir R dans le produit final, on estérifie éventuellement le groupe hydroxy en 17 avant ou après avoir éliminé le groupe protecteur.

**Revendication pour l'Etat contractant: AT**

Procédé de préparation de méthylène-11 stéroides répondant à la formule générale I

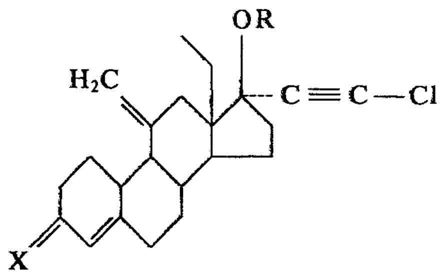

(I)

dans laquelle

R représente un atome d'hydrogène ou un radical acyle et
X représente un atome d'oxygène ou deux atomes d'hydrogène,

procédé caractérisé en ce qu'on fait réagir un méthylène-11 oxo-17 stéroide répondant à la formule générale II

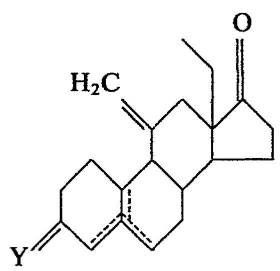

(II)

dans laquelle

Y représente un groupe oxo libre ou protégé par un groupe hydrolysable en milieu acide ou représene deux atomes d'hydrogène et
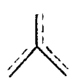 représente une double liaison en position 4,5, 5,6 ou 5,10 ou deux doubles liaisons partant de la position 3 et de la position 5,

par des méthodes connues, avec le dichloro-1,2 éthylène et une solution éthérée d'un alkyl-lithium, de manière à obtenir un composé de formule générale I dans lequel R représente un atome d'hydrogène, on hydrolyse un groupe protcteur de la fonction cétonique en 3 dans le cas où l'on a préalablement introduit un tel groupe et, suivant la signification que doit avoir R dans le produit final, on estérifie éventuellement le groupe hydroxy en 17 avant ou après avoir éliminé le groupe protecteur.